# EUROPÄISCHE PATENTANMELDUNG

(11) **EP 4 001 293 A1**
(43) Veröffentlichungstag der Anmeldung: **25.05.2022**
(21) Anmeldenummer: 20209127.8
(22) Anmeldetag: 23.11.2020
(51) Int. Cl.: C07K 5/10, A61K 38/07, A61P 15/08, A61P 19/10, A61P 5/26

(54) **TETRAPEPTID UND DESSEN VERWENDUNG**

(71) Anmelder: Justus-Liebig-Universität Gießen, 35390 Gießen (DE)
(72) Erfinder: Scheiner-Bobis, Georgios, 35510 Butzbach (DE); Bulldan, Ahmed, 35394 Gießen (DE); Malviya, Viveka Nand, 37077 Göttingen (DE)
(74) Vertreter: Stumpf, Peter

(57) **Zusammenfassung**

Der Gegenstand der Erfindung betrifft ein an den ZIP-9-Rezeptor bindendes Tetrapeptid zur Behandlung von Osteoporose, myodegenerativen Erkrankungen und männlicher Infertilität. Weiterhin betrifft die Erfindung die Verwendung des Tetrapeptides als Nahrungs- und Futterergänzungsmittel und die in vitro Anwendung zur Züchtung von Muskelmasse (in vitro Fleischproduktion).

SEQUENCE LISTING

<110> Justus-Liebig-Universität Gießen

<120> Tetrapeptid und dessen Verwendung

<130> TM1095

<160> 6

<170> BiSSAP 1.3.6

<210> 1
<211> 4
<212> PRT
<213> Artificial Sequence

<220>
<223> bindet am membrangebundenen Androgenrezeptor ZIP-9

<400> 1
Ile Ala Pro Gly
1

<210> 2
<211> 4
<212> PRT
<213> Artificial Sequence

<220>
<223> bindet am membrangebundenen Androgenrezeptor ZIP-9

<400> 2
Gly Val Ser Gly
1

<210> 3
<211> 4
<212> PRT
<213> Artificial Sequence

<220>
<223> bindet am membrangebundenen Androgenrezeptor ZIP-9

<400> 3
Gly Val Val Gly
1

<210> 4
<211> 4
<212> PRT
<213> Artificial Sequence

<220>
<223> bindet am membrangebundenen Androgenrezeptor ZIP-9

<400> 4
Pro Gln Thr Gly
1

<210> 5
<211> 4
<212> PRT
<213> Artificial Sequence

<220>
<223> bindet am membrangebundenen Androgenrezeptor ZIP-9

<400> 5
Ser Gly Asn Leu
1

<210> 6
<211> 4
<212> PRT
<213> Artificial Sequence

<220>
<223> bindet am membrangebundenen Androgenrezeptor ZIP-9

<400> 6
Gln Ala Pro Gly
1

## Beschreibung

### Tetrapeptid und dessen Verwendung

Die vorliegende Erfindung betrifft ein Tetrapeptid, d. h. ein Peptid aus vier Aminosäuren, sowie dessen Verwendung als Nahrungs- und Futterergänzungsmittel sowie als medizinisches Mittel zur Behandlung von Osteoporose, myodegenerativen Erkrankungen, d.h. Myopathien wie zum Beispiel Muskeldystrophie und insbesondere Muskelatrophie (=Muskelschwund, Amyotrophie), und männlicher Infertilität. Dem Fachmann sind die grundsätzlichen Charakteristika von Peptiden, z. B. deren chemische Strukturen und Eigenschaften, bekannt.

### Stand der Technik

Osteoporose, myodegenerative Erkrankungen sowie männliche Infertilität werden oftmals durch medikamentöse Gaben von Testosteron oder Testosteron-Derivaten behandelt. Diese Art der Behandlung wirkt über den klassischen nukleären Androgen-Rezeptor (AR) und führt zu einer Vielzahl ungewollter Nebenwirkungen, beispielsweise die ungewollte Stimulation des Wachstums der Körperbehaarung (Hirsutismus), Bluthochdruck, Störungen des Fettstoffwechsels, der Blutgerinnung und des Gefäßsystems. Zumindest einige der Nebenwirkungen von Testosteron beruhen darauf, dass Testosteron in die Zelle eindringt, dort in seine aktive Form, das Dihydrotestosteron (5a-Dihydrotestosteron) überführt wird, und anschließend seine Hormonwirkung entfaltet. Weitere Behandlungsmöglichkeiten gegen Osteoporose und myodegenerative Erkrankungen sind etwa die Applikation von Bisphosphonaten oder selektiven Östrogen-Rezeptor-Modulatoren. Beide Wirkstoffklassen hemmen die Knochenresorption. Auch Parathormon, ein Peptidhormon mit 84 Aminosäuren und seine Analoga werden eingesetzt.

All diese Behandlungsmöglichkeiten weisen unterschiedliche gravierende Nachteile auf. Bisphosphonate werden z. B. schlecht resorbiert (vom Körper aufgenommen) und bilden zudem mit Calcium unlösliche Komplexe. Das heißt, die Dosis muss sehr hoch angesetzt werden, damit ausreichend Wirkstoff vom Körper aufgenommen wird, gleichzeitig bindet die hohe Wirkstoffkonzentration aber auch Calciumionen, was für die Knochenbildung sehr nachteilig ist.

Parathormon weist die üblichen Schwierigkeiten in der Herstellung und Handhabung von Peptidhormonen auf (z. B. Denaturierung) und ist daher gegenüber dem erfindungsgemäßen Tetrapeptid sehr viel problematischer in der Herstellung, Handhabung (Lagerung) und Applikation. Die daraus zwangsläufig resultierenden Vorteile des erfindungsgemäßen Tetrapeptides gegenüber Parathormon und seinen Analoga sind dem Fachmann bereits aus der jeweiligen Anzahl der Aminosäuren-Bausteine (4 versus 84) ohne weiteres ersichtlich.

### Aufgabe

Aufgabe der vorliegenden Erfindung ist es, die im Stand der Technik bestehenden Nachteile bei der Behandlung der Osteoporose, myodegenerativen Erkrankungen sowie der männlichen Infertilität, insbesondere die zahlreichen mit den bekannten Behandlungsverfahren verbundenen Nebenwirkungen zu vermeiden und ein geeignetes Mittel zur Behandlung bereitzustellen.

### Lösung der Aufgabe

Diese Aufgabe wird erfindungsgemäß gelöst durch die Bereitstellung eines Tetrapeptides und dessen Verwendung gemäß der Ansprüche.

Neben dem bereits bekannten Androgen-Rezeptor (AR), der als NRS 34 zur Superfamilie der nukleären Rezeptoren zählt, ist seit Kurzem ein weiterer Androgen-rezeptor (ZIP-9) bekannt, der sich natürlicherweise in der Zellmembran befindet. ZIP-9 besitzt eine Testosteronbindungsstelle, die sich auf der extrazellulären Seite befindet und somit von außen zugänglich ist. ZIP-9 findet man in den verschiedensten Zellen, somit auch in knochenbildenden Osteoblasten, in muskelbildenden Myoblasten und auch in den Blut-Hoden-Schranke-bildenden Sertoli-Zellen. Testosteron kann an der Bindungsstelle von ZIP9 andocken und in Osteoblasten die Ossifikation (Knochenbildung) und die Umwandlung der Osteoblasten zu Osteozyten (Knochenzellen) stimulieren. Testosteron bindet auch an den ZIP-9-Rezeptor von Myoblasten und stimuliert dort die Ausbildung von Myotuben (Muskelfasern). Fatal für die therapeutischen Anwendungen von Testosteron gegen Osteoporose, myodegenerative Erkrankungen sowie der männlichen Infertilität ist aber seine parallel dazu einsetzende Wirkung an den klassischen nukleären Androgen-Rezeptor (AR), da diese die bekannten Nebenwirkungen verursacht und daher nicht erwünscht ist.

Die Erfinder hatten es sich daher zur Aufgabe gesetzt, Wirkstoffe zu entwickeln, die sehr selektiv nur an den ZIP-9-Rezeptor binden und dort ihre therapeutische Wirkung entfalten, ohne gleichzeitig auch am klassischen nukleären Androgen-Rezeptor (AR) zu binden.
Ausgehend von molekültheoretischen Berechnungen (z.B. ab initio-Struktur- und - Strukturdynamik-Berechnungen) wurden mehrere Peptide errechnet, welche innerhalb der Androgen-Bindungsstelle von ZIP-9 binden können. Sechs Peptide mit den besten Bindungseigenschaften wurden hergestellt und weiter analysiert indem sie auf ihre Bindung und Wirksamkeit hin untersucht wurden. Es handelt sich durchgehend um Tetrapeptide (kurze Peptide aus 4 Aminosäuren) welche an der Androgen-Bindungsstelle des Androgenrezeptors ZIP-9 binden und dadurch in Osteoblasten die Ossifikation, also die Umwandlung der Osteoblasten zu Osteozyten stimulieren und in Myoblasten die Ausbildung von Muskelfasern. Die sechs errechneten und synthetisierten Tetrapeptide sind in Tabelle 1 zusammengefasst. Sie binden sehr selektiv in der Androgen-Bindungsstelle des ZIP-9 und vermitteln über diesen Rezeptor ihre androgene Wirkung. Da die Androgen-Bindungsstelle des ZIP-9 auf der jeweiligen Zelloberfläche liegt, lösen die erfindungsgemäßen Tetrapeptide androgene Effekte aus, ohne die unerwünschten Wirkungen des klassischen Androgen-Rezeptors (AR) hervorzurufen, der sich im Zellkern befindet. Die sechs Tetrapeptide binden nur an den ZIP-9-Rezeptor, der natürlicherweise in der Zellmembran lokalisiert, also membrangebunden ist und lösen über den androgenen Wirkmechanismus des ZIP-9-Rezeptors die gleiche positive Wirkung aus. Der Androgen-Rezeptor ZIP-9 befindet sich nicht nur an Zellmembranen von Osteoblasten oder Myoblasten sondern natürlicherweise auch in den Zellmembranen zahlreicher weiterer Zellarten, so dass die sechs Tetrapeptide weitere therapeutische Effekte aufweisen. So stimulieren sie die Bildung der Blut-Hoden-Schranke und sind dadurch auch für die Behandlung von infertilen Männern und männlichen Tieren insbesondere Säugetieren geeignet.
Die sechs Tetrapeptide (Seq ID 1-6) weisen somit knochen- und muskelbildende Wirkungen, sowie Blut-Hoden-Schranke stimulierende Wirkung auf, sowohl im tierischen als auch im menschlichen Organismus. Sie werden erfindungsgemäß als pharmazeutische Wirkstoffe ("API" - active pharmaceutical ingredients) verwendet. Das oft eingesetzte Testosteron und die Testosteronanaloga, sowie die weiteren im Stand der Technik bekannten Wirkstoffe, die allesamt umfangreiche Nebenwirkungen aufweisen, können daher durch die sechs Tetrapeptide in der Therapie von Osteoporose, myodegenerativen Erkrankungen, wie z.B. Muskelschwund und männlicher Infertilität ersetzt werden.
Wie die Ausführungsbeispiele zeigen, wird besonders vorteilhaft durch Tetrapeptid 1 (Seq ID1) in Osteoblasten die Knochenbildung stimuliert. In Myozyten, die zwar den Androgen-Rezeptor ZIP- 9 aufweisen, aber keinen klassischen Androgen-Rezeptor (AR) exprimieren, wird durch die Tetrapeptid 1 (Seq ID1) die Muskelfaser-Bildung besonders gut stimuliert. Die Ausführungsbeispiele zeigen Ergebnisse von Tetrapeptid 1, Tetrapeptid 2 und Tetrapeptid 3, die Ergebnisse von Tetrapeptid 4, Tetrapeptid 5 bis Tetrapeptid 6 werden hier nicht gezeigt, sie entsprechen aber den Ergebnissen von Tetrapeptid 2.
Tabelle 1 zeigt die im Rahmen der Erfindung eingesetzten Tetrapeptide Seq ID 1-6 insbesondere das erfindungsgemäße Tetrapeptid Seq ID 1. Die in der Tabelle und weiter nachfolgend zur Beschreibung der Tetrapeptide verwendete 1-Buchstaben-Codierung für Aminosäuren ist dem Fachmann bekannt, so dass diese Angaben die Tetrapeptide (Seq ID 1-6) vollständig und eindeutig beschreiben, einschließlich der chemischen Verknüpfung der einzelnen Aminosäuren in den Tetrapeptiden sowie der C- und N-terminalen Enden der Tetrapeptide. Die Tetrapeptide umfassen die Aminosäuren Alanin (A), Isoleucin (I), Prolin (P), Glycin (G), Valin (V), Serin (S), Glutamin (Q), Threonin (T), Asparagin (N), Leucin (L), wobei jedes Tetrapeptid mindestens ein Glycin (G) enthält. Dies ist entscheidend für die Struktur, Stabilität und die Größe der Tetrapeptide. Glycin (G) ist eine optimale Aminosäure, weil sie den Tetrapeptiden (Seq ID 1-6) optimale sterischen Eigenschaften gibt, sodass eine optimale Verabreichung möglich ist.

**Tabelle 1: Aminosäuresequenzen der Tetrapeptide**

| Peptid-1 Seq ID 1 | Peptid-2 Seq ID 2 | Peptid-3 Seq ID 3 | Peptid-4 Seq ID 4 | Peptid-5 Seq ID 5 | Peptid-6 Seq ID 6 |
|---|---|---|---|---|---|
| IAPG | GVSG | GWG | PQTG | SGNL | QAPG |

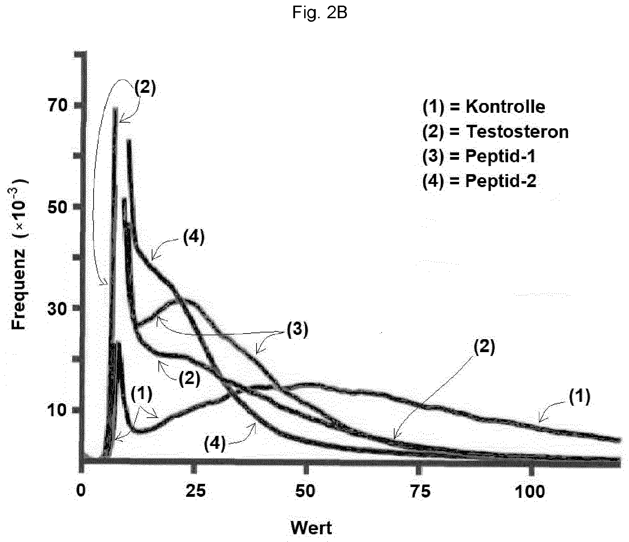

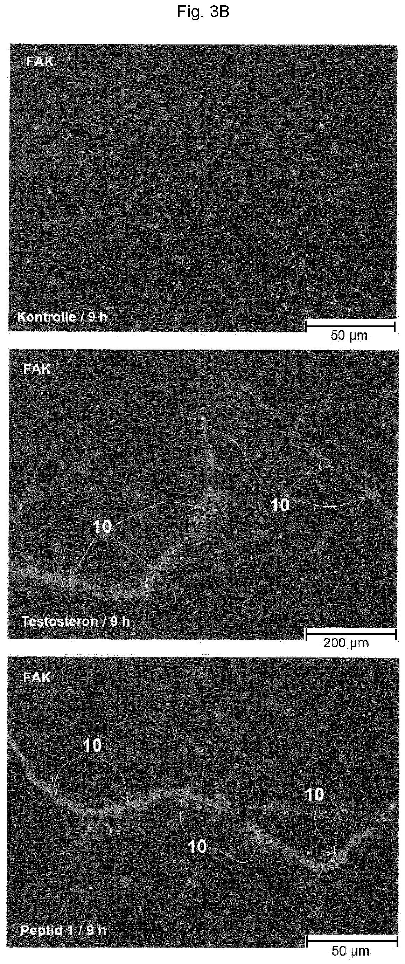

Die Ausführungsbeispiele zeigen, dass die Tetrapeptide Seq ID 1-6 wenigstens die gleiche Wirksamkeit aufweisen wie Testosteron und die Testosteronanaloga. Dabei weisen sie als Tetrapeptide jedoch nicht die hormonbedingten Nebenwirkungen auf, da sie nur am membrangebundenen Androgenrezeptor ZIP-9 binden und nicht im Zellkern am klassischen Androgenrezeptor (AR). Eine besonders positive Wirkung kommt dem erfindungsgemäßen Tetrapeptid 1 Seq ID 1 zu.
Die Wirksamkeit der erfindungsgemäßen Tetrapeptide Seq ID 1-6 zur Behandlung der Osteoporose, myodegenerativen Erkrankungen (zum Beispiel Muskeldystrophie, insbesondere jedoch Muskelatrophie) sowie der männlichen Infertilität wird experimentell in Zellkultur in einem charakteristischen Zellmodell gezeigt.
Zur Verabreichung an Menschen und Tiere wird mindestens ein erfindungsgemäße Tetrapeptid als medizinisches Mittel in einer pharmazeutisch akzeptablen Komposition als Arzneimittel formuliert. Das medizinische Mittel wird zur Behandlung der Osteoporose, myodegenerativen Erkrankungen sowie der männlichen Infertilität bei Menschen und Tieren eingesetzt. Es löst dabei keine übermäßige Toxizität, Irritationen oder allergische Reaktionen aus. Bevorzugt wird das medizinische Mittel umfassend mindestens ein erfindungsgemäßes Tetrapeptid Menschen oder Tieren in Form entweder oral, intramuskulär, intraossär, rektal, parenteral, intravenös, subkutan, intracisternal, intravaginal, intraperitoneal, intrathekal, intravasculär, lokal (Puder, Salbe oder Tropfen), in Sprayform oder als Pflaster verabreicht. Dosierungsformen für die örtliche Administration des medizinischen Mittels dieser Erfindung schließen Salben, Puder, Sprays oder Inhalationsmittel ein. Das mindestens eine erfindungsgemäße Tetrapeptid als medizinisches Mittel wird unter sterilen Bedingungen mit einem physiologisch akzeptablen Trägerstoff und möglichen Konservierungsmitteln, Puffern oder Treibmitteln, je nach Bedarf, vermischt. Ein Konservierungsmittel ist eine Chemikalie, die zugesetzt wird, um die Zersetzung zu verhindern. Es hilft, das medizinische Mittel gegen Bakterien- und Pilzbefall oder Abbau durch Enzyme zu schützen, indem es das Wachstum von Bakterien oder Pilzen verhindern und/oder die Zersetzung des Mittels verlangsamt.
Bevorzugt wird das medizinische Mittel dieser Erfindung als Lyophilisat hergestellt. Bevorzugt wird das medizinische Mittel dieser Erfindung oral verabreicht. Da das darin enthaltene mindestens eine Tetrapeptid sehr klein ist, wird es von proteolytischen Enzymen nicht so rasch abgebaut und kann seine Wirksamkeit am ZIP-9 Rezeptor sehr selektiv und sehr spezifisch entfalten.
Die Tetrapeptide Seq ID 1-6 werden gemäß Verfahren, die der Fachmann kennt, einfach und mit geringen Kosten hergestellt. Sie weisen in lyophilisierter Form eine lange Lagerungsbeständigkeit und hohe Aktivität auf. Die Lagerung ist bei Raumtemperatur möglich. Ein Vorteil ist, dass aufgrund der hohen Spezifität nur geringe Mengen verabreicht werden müssen, um an den ZIP-9 Rezeptor zu binden und dort die positiven Effekte auszulösen.
In einem besonderen Ausführungsbeispiel wird das Tetrapeptid Seq ID 1 verkapselt, damit es im Magen-Darm-Trakt nicht abgebaut (säure-hydrolysiert), sondern als Tetrapeptid in den Blutkreislauf aufgenommen wird. Dazu werden z.B. mesoporöse Silicapartikel verwendet, die die Bioverfügbarkeit verlängern. Alternativ werden an das Tetrapeptid Polymere angehängt, wie z.B. Polyethlenglycolgruppen (PEGylierung). Diese Polymere sind bekannt für ihre geringe Adsorption an Plasmaproteinen, was die Wirkstoffe länger stabil im Blutkreislauf hält und sie an zelluläre Oberflächenrezeptoren gelangen lässt. Diese Maßnahmen schützen auch - und verlängern die Bioverfügbarkeit - der Tetrapeptide Seq ID 2-6. Der Abbau der Tetrapeptide durch Verdauungsenzyme wird verlangsamt, indem D-Aminosäuren statt L-Aminosäuren verwendet werden.
Die Art der Dosierung des medizinischen Mittels dieser Erfindung wird vom behandelnden Arzt entsprechend den klinischen Faktoren bestimmt. Es ist dem Fachmann bekannt, dass die Art der Dosierung von verschiedenen Faktoren wie z.B. Körpergröße, Gewicht, Körperoberfläche, Alter, Geschlecht oder der allgemeinen Gesundheit des Patienten abhängig ist, aber auch von dem speziell zu verabreichenden Mittel, der Dauer und Art der Verabreichung und von anderen Medikamenten, die möglicherweise parallel verabreicht werden.
Das medizinische Mittel dieser Erfindung wird zur Therapie der Osteoporose, myodegenerativen Erkrankungen sowie der männlichen Infertilität angewendet.
Als myodegenerative Erkankungen gelten alle Krankheiten mit Knochenabbau assoziiert sind, wie z.B. Muskelschwund und Muskelatrophie sowie auch Muskeldystrophie.
Die besondere Wirksamkeit von Tetrapeptid Seq ID 1 umfasst die Stimulierung der Ossifikation durch die Aktivierung von Osteosarkomzellen SAOS-2-Zellen. Experimentell belegt wird diese der Stimulation der Ossifikation in SAO-2-Zellen durch Einfärbung der Zellen mit Alizarinrot. Der quantitative Messwert zur Beschreibung der Stimulation ist dabei die Farbstärke der mit dem Tetrapeptid Seq ID 1 behandelten Zellen bei mikroskopischer Betrachtung im direkten Vergleich mit einer gleichbehandelten Kontrollprobe ohne Tetrapeptid. Die jeweils unter gleichen Einfärbe- und Messbedingungen gemessene Farbstärke in mit dem erfindungsgemäßen Tetrapeptid-behandelten Zellen beträgt wenigstens den dreifachen Wert der Farbstärke in der gleich behandelten Kontrollprobe. Dem Fachmann ist aus dem Stand der Technik bekannt, wie das Einfärben der behandelten Zellen und der Kontrollzellen, und auch die Auswahl des für die Messung verwendeten Farbtons und die Bestimmung der Farbstärke vorzunehmen ist, um miteinander vergleichbare Messwerte zu erhalten.
Die Wirksamkeit der erfindungsgemäßen Tetrapeptid Seq ID 1 umfasst weiterhin die Reduzierung der Expression der Alkalischen Phosphatase (AP) in Zellen. Die Alkalische Phosphatase (AP, ALP, knochenspezifische Ostase) ist ein Enzym, das an vielen Stoffwechselgeschehen des menschlichen oder tierischen Körpers beteiligt ist. Es gibt verschiedene Gruppen von alkalischen Phosphatasen. Sie befinden sich unter anderem in den Knochenzellen und im Lebergewebe. Bei einem erhöhten Stoffwechsel im Knochen, z.B. wenn Osteoklasten abgebaut werden, wird die knochenspezifische ALP vermehrt freigesetzt und ist daher vermehrt im Blut nachweisbar. Sie ist also ein Biomarker für den Knochenstoffwechsel. Die Alkalische Phosphatase wirkt dephosphorylierend, d.h. der Knochenmasse wird Phosphat entzogen, wodurch die Knochendichte sinkt. Eine Absenkung der Konzentration und/oder der Aktivität der ALP wirkt daher der Osteoporose entgegen und zeigt gleichzeitig an, dass Osteoblasten sich zu Osteozyten weiter entwickelt haben. Damit wird durch Verabreichung der erfindungsgemäßen Tetrapeptide sowohl der Knochenabbau gehemmt als auch die Knochensubstanz stabilisiert. Experimentell belegt wird dies durch die Quantifizierung der Reduktion der Expression der ALP in SAOS-2 Osteoblasten. Dies erfolgt durch quantitative Auswertung der Messung der Expression der Alkalischen Phosphatase in den Zellen. Dabei ist der quantitative Messwert zur Beschreibung der Reduktion der Expression der ALP die Häufigkeit (d. h. die "Frequenz", beispielsweise in Fig. 2B auf der Ordinaten aufgetragen), mit der mindestens ein bestimmter Wert der Fluoreszenzintensität gemessen wird. Die Werte der Fluoreszenzintensität sind beispielsweise als "Wert" auf der Abszisse der Fig. 2B aufgetragen. Die Häufigkeit von mindestens einem bestimmten Wert der in den mit dem Tetrapeptid Seq ID 1 behandelten Zellen gemessenen Werte im direkten Vergleich mit der Häufigkeit des gleichen mindestens einen Wertes, der in Tetrapeptid-freien aber ansonsten gleich behandelten Kontrollzellen gemessen wird, beträgt dabei ab dem Wert 65 höchstens 50 % der in den gleich behandelten Kontrollzellen gemessenen Häufigkeit ab dem Wert 65.
Die besondere Wirksamkeit von Tetrapeptid Seq ID 1 umfasst weiterhin die die Stimulierung der Fokalen Adhäsionskinase (FAK) in Myoblasten. Experimentell erfolgt die Quantifizierung der Stimulierung der Fokalen Adhäsionskinase (FAK) in Zellen L6-Myoblasten durch Fluoreszenzmessung. Diese Zellen exprimieren von Natur aus keinen klassischen Androgenrezeptor sondern nur ZIP9. Der quantitative Messwert zur Beschreibung der Stimulierung der Fokalen Adhäsionskinase, bzw. deren Expression, ist die Fluoreszenzintensität der mit dem Tetrapeptid behandelten Zellen bei mikroskopischer Betrachtung im direkten Vergleich mit einer gleichbehandelten, aber tetrapeptidfreien, Kontrollprobe. Die gemessene Fluoreszenzintensität in den Tetrapeptid behandelten Zellen beträgt wenigstens den dreifachen Wert der Fluoreszenzintensität verglichen mit einer gleich behandelten Kontrollprobe ohne Tetrapeptid.
Die Stimulierung der FAK ist Voraussetzung für die Umwandlung von Myoblasten zu Myotuben (Muskelfasern) welche aus miteinander verschmolzenen Myoblasten-Zellen entstehen. Aus der Abbildung Fig. 3B ist ersichtlich, dass Tetrapeptid Seq ID 1 die Muselfaserbildung stimuliert und somit für die Behandlung von Muskelschwund und anderen degenerativen Muskelerkrankungen geeignet ist. Außerdem stimuliert es auch die Expression von Myogenin. Myogenin ist ein Transkriptionsfaktor in Wirbeltieren, der an der Aktivierung muskelspezifischer Gene beteiligt ist. Insbesondere tritt Myogenin in Regenerationsphasen in Skelettmuskeln auf und spielt eine Rolle bei der Bildung von Skelettmuskelfasern während bestimmter Stadien der embryonalen Entwicklung. Myogenin ist daher ein Biomarker für die Regeneration von Skelettmuskeln. Experimentell erfolgt die Quantifizierung der Stimulation der Bildung von Myogenin in L6-Myoblasten durch Fluoreszenzmessung. Der quantitative Messwert zur Beschreibung der Stimulation der Bildung von Myogenin in der Zelle ist die Fluoreszenzintensität der mit dem erfindungsgemäßen Tetrapeptid behandelten Zellen bei mikroskopischer Betrachtung im direkten Vergleich mit einer gleichbehandelten Kontrollprobe ohne Tetrapeptid. Die gemessene Fluoreszenzintensität beträgt wenigstens den dreifachen Wert der Fluoreszenzintensität in der Kontrollprobe.

Die besondere Wirksamkeit von Tetrapeptid Seq ID 1 umfasst weiterhin die Stimulierung der Expression von Zonula Occludens-1 (ZO-1), Claudin-1 und Claudin-5 in Sertoli-Zellen.
ZO-1 ist ein Protein, das nur auf der zytoplasmatischen Seite der Tight Junctions von Zellen vorkommt und dort mit Proteinen Occludin und den verschiedenen Claudinen, darunter auch Claudin-1 und Claudin-5, sowie den Aktin-Filamenten des Cytoskeletts interagiert. ZO verbindet die Tight Junctions mit dem Cytoskelett und trägt zur deren Stabilität bei. Es spielt eine Rolle als Gerüstprotein, das Tight Junction Proteine, bei denen es sich um faserartige Strukturen innerhalb der Lipiddoppelschicht handelt, vernetzt und am Aktin-Cytoskelett verankert. Es sind drei Isoformen ZO-1, ZO-2 und ZO-3 bekannt. ZO-1 trägt wesentlich zur Ausbildung und Stabilität der Tight Junctions zwischen den Sertoli-Zellen der männlichen Gonaden bei.

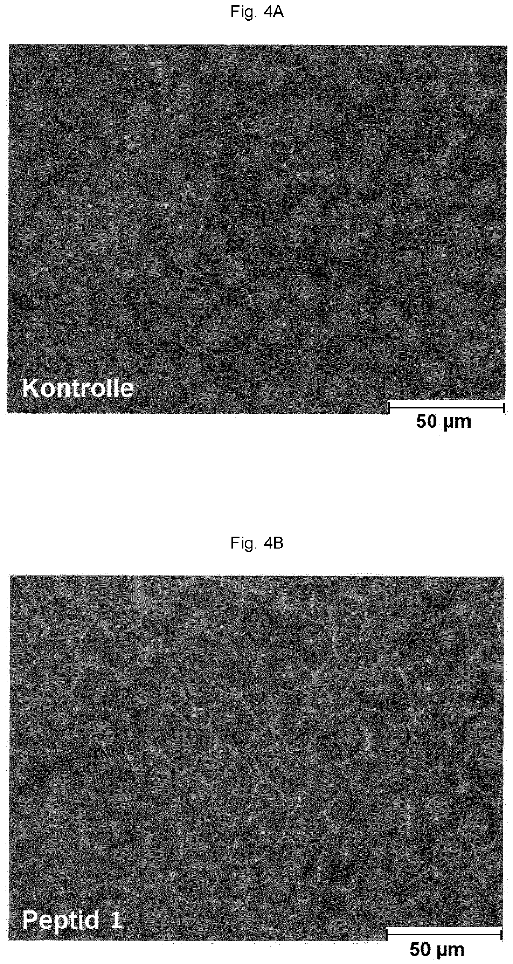

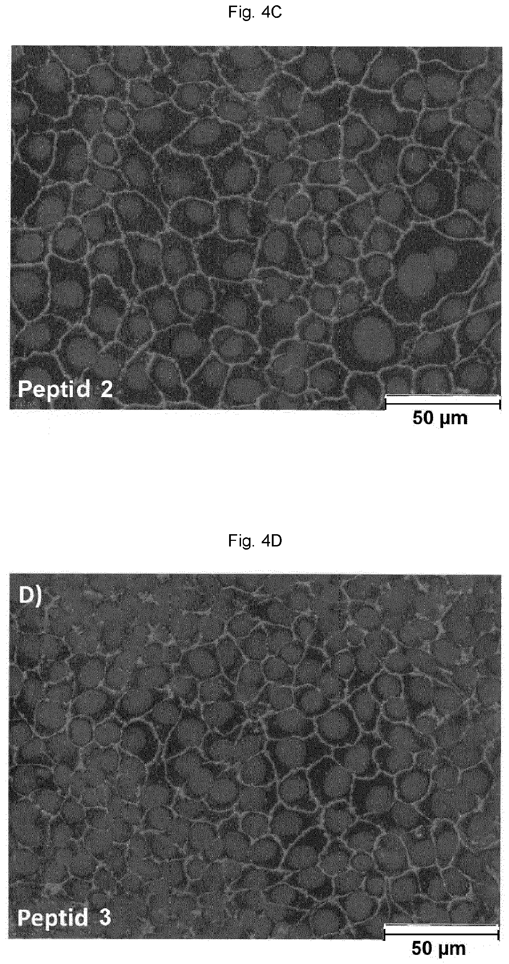

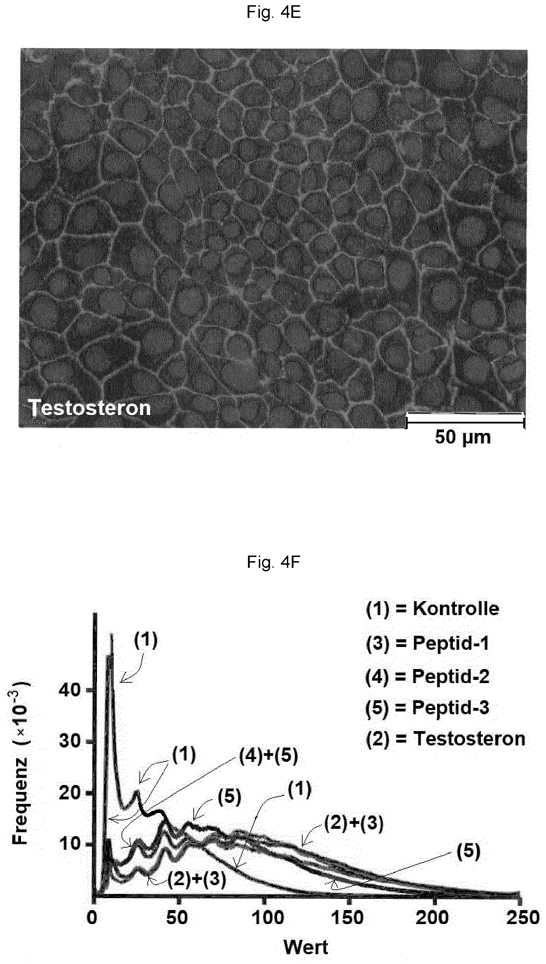

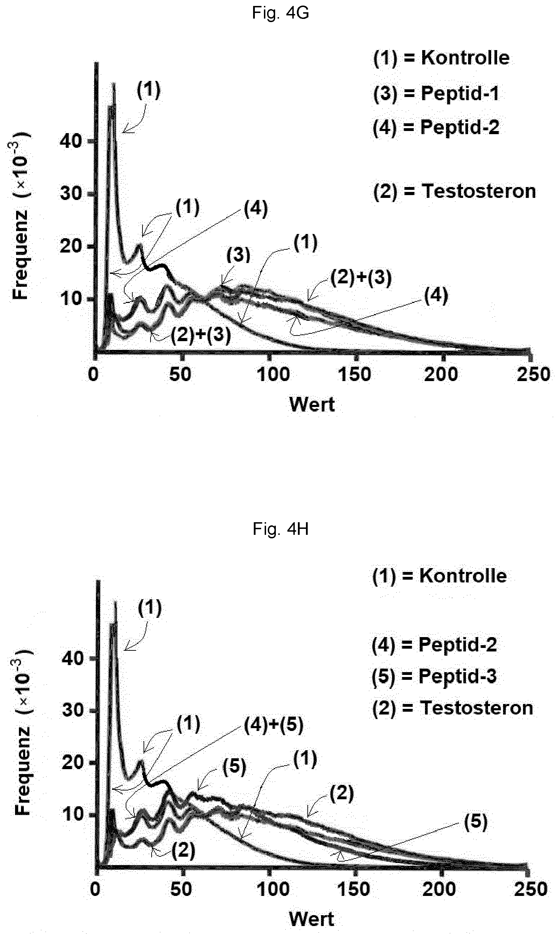

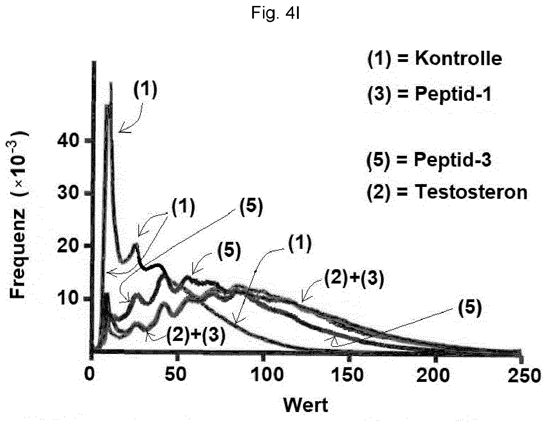

Experimentell erfolgt die Quantifizierung der Stimulation der Bildung von ZO-1 in 93RS2 Sertoli-Zellen durch quantitative Auswertung der Messung der Expression von ZO-1. Der quantitative Messwert zur Beschreibung der Stimulation der Expression von ZO-1 ist die Häufigkeit (Frequenz), mit der mindestens ein bestimmter Wert ("Wert") der Fluoreszenzintensität gemessen wird. Die Bedeutung der Begriffe "Häufigkeit"/"Frequenz" und "Wert"/"Wert der Fluoreszenzintensität" ist dabei analog der Bedeutung im Zusammenhang mit der Alkalischen Phosphatase und bezieht sich hierbei auf Fig. 4F bzw. Fig. 4G bzw. Fig. 4H bzw. Fig. 4I. Die Häufigkeit des mindestens einen bestimmten Wertes der in den mit dem erfindungsgemäßen Tetrapeptid behandelten Zellen gemessenen Werte im direkten Vergleich mit der Häufigkeit des mindestens einen gleichen Wertes, die in Tetrapeptid-freien aber ansonsten gleich behandelten Kontrollzellen gemessen wird, beträgt ab dem Wert 70 mindestens 120 % der in den gleich behandelten Kontrollzellen gemessenen Häufigkeit ab dem Wert 70.

Die besondere Wirksamkeit von Tetrapeptid Seq ID 1 umfasst weiterhin die Stimulierung der Bildung von Claudin-1 und Claudin-5 in Zellen. Claudine sind kleine Transmembranproteine, die in vielen Organismen, Menschen und Tieren vorkommen und in ihrem Aufbau sehr ähnlich sind. Sie haben entscheidende Funktionen zur Bildung einer funktionierenden Barriere in verschiedenen Epithelien. Es gibt zahlreiche Isoenzyme von Claudinen, die weitgehend ubiquitär exprimiert sind und Grundfunktionen für die Bildung der Tight-Junction-Bänder übernehmen.
Caudin-1 und Claudin-5 sind spezialisierte Claudine, die an der Blut-Hoden-Schranke der männlichen Gonaden exprimiert werden. Ihre Beteiligung an der Ausbildung und Aufrechterhaltung der Tight Junctions an der Blut-Hoden-Schranke schützt die männlichen Keimzellen vor Angriffen des eigenen Immunsystems. Somit gehen Defekte der Blut-Hoden-Schranke immer mit Infertilität einher.
Durch die Steigerung der Expression von Claudin-1 und/oder Claudin-5, ggf. zusammen mit der Stimulierung der Expression von ZO-1 durch die erfindungsgemäßen Tetrapeptide ist es möglich, bei männlicher Infertilität diese Krankheitssymptome zu beseitigen um Fertilität zu regenerieren. Experimentell erfolgt die Quantifizierung der Stimulation der Bildung von Claudin-1 und/oder Claudin-5 in der Zelle durch Fluoreszenzmessung. Der quantitative Messwert zur Beschreibung der Stimulation der Bildung von Claudin-1 und/oder Claudin-5 in 93RS2 Sertoli-Zellen ist die Fluoreszenzintensität der mit dem Tetrapeptid behandelten Zellen bei mikroskopischer Betrachtung im direkten Vergleich mit einer gleichbehandelten Kontrollprobe ohne Tetrapeptid. Die gemessene Fluoreszenzintensität der mit dem Tetrapeptid behandelten Zellen beträgt wenigstens den dreifachen Wert der Fluoreszenzintensität in der Kontrollprobe.

Weiterhin umfasst der Gegenstand der Erfindung die Verwendung mindestens eines der erfindungsgemäßen Tetrapeptide als Nahrungsergänzungsmittel.
Weiterhin umfasst der Gegenstand der Erfindung die Verwendung mindestens eines der erfindungsgemäßen Tetrapeptide als Futterergänzungsmittel. Damit kann es in der Tierhaltung zur Mast insbesondere zum Aufbau von Muskelmasse eingesetzt werden. Dies hat den Vorteil, dass dieses Futterergänzungsmittel den Einsatz von Antibiotika als Mastmittel in der Tierhaltung zumindest teilweise überflüssig macht. Somit kann die Ausbildung von Antibiotikaresistenzen infolge des Einsatzes von Antibiotika in der Tiermast zumindest deutlich reduziert werden.

Weiterhin umfasst der Gegenstand der Erfindung die Verwendung mindestens eines der erfindungsgemäßen Tetrapeptide zur in vitro Züchtung von Muskelmasse. Damit können Muskelzellen in vitro zu größeren Muskeleinheiten wachsen. Dies findet Einsatz entweder im therapeutischen Sinne als Ersatz für regenerative Zwecke bei verschiedenen Muskelerkrankungen oder als Grundlage für die Herstellung von Fleisch zum Verzehr.

### Ausführungsbeispiele

Bei allen Ausführungsbeispielen wird als positive Kontrolle Testosteron in vergleichbarer, d.h. physiologischer bzw. therapeutischer Konzentration (entsprechend den im Stand der Technik bekannten Anwendungen von Testosteron) eingesetzt.

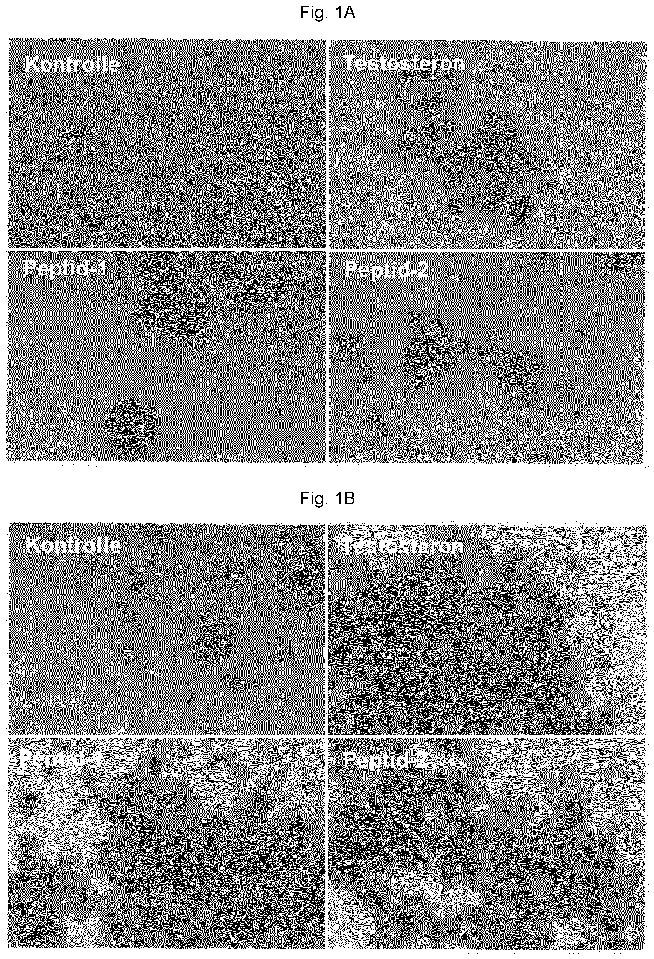

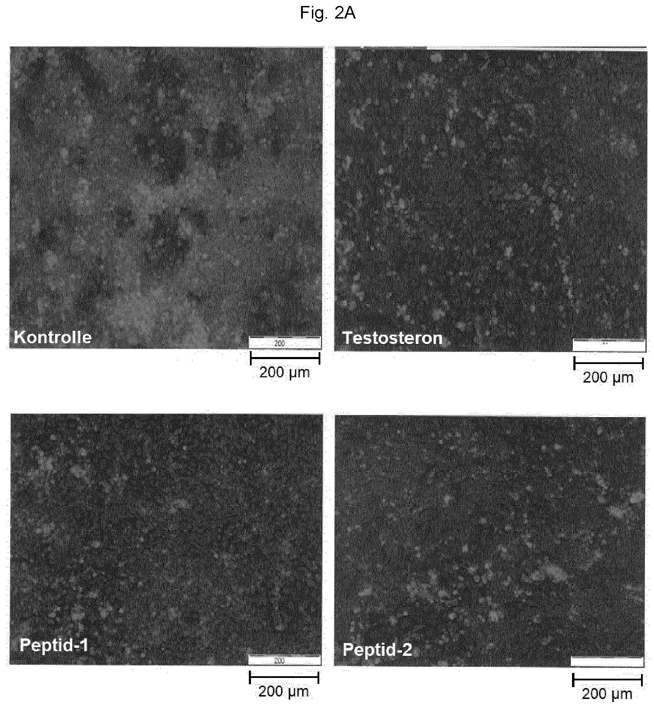

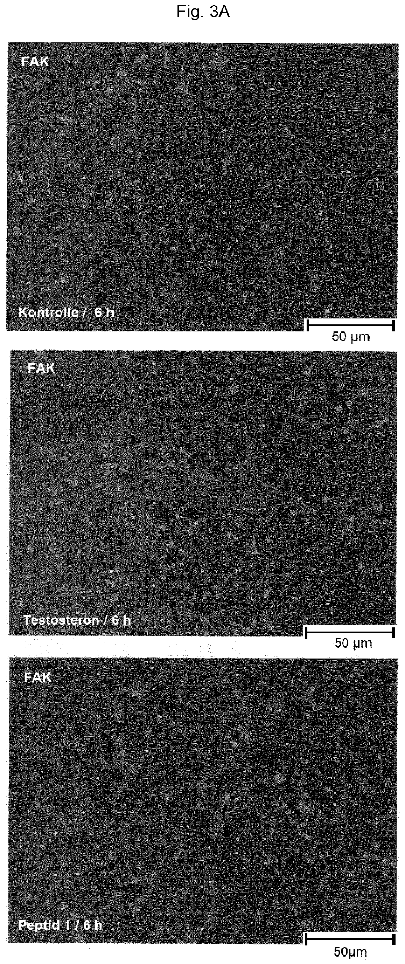

Die Figuren 1A und 1B zeigen die Stimulierung der Ossifikation in SAOS-2-Zellen durch die Tetrapeptide (Peptid-1 und Peptid-2) nach jeweils 4 Tagen (Fig. 1A) und 11 Tagen (Fig. 1B) Inkubation der SAOS-2-Zellen mit den Tetrapeptiden. Der Nachweis der Stimulierung der Ossifikation erfolgt hierbei durch Einfärbung mit Alizarinrot nach Standardvorgabe. Die Durchführung des Färbevorganges erfolgt bei allen Proben (Referenz, Vergleichsprobe mit Testosteron und Tetrapeptid) in gleicher Weise, so dass die Farbstärke (erkennbar in der photographischen Darstellung in Fig. 1A und Fig. 1B anhand der Grauwerte in der jeweiligen Abbildung) als Maß für die Stimulierung dient (je dunkler der Grauwert, umso stärker die Stimulation zur Ossifikation).
Die Wirksamkeit der Tetrapeptide (Peptid-1 und Peptid-2) wird weiterhin anhand der Expression der Alkalischen Phosphatase (ALP) nachgewiesen, wobei auch hierbei Testosteron als Referenzsubstanz verwendet wird. Die Alkalische Phosphatase wirkt dephosphorylierend, d.h. der Knochenmasse wird Phosphat entzogen, wodurch die Knochendichte sinkt. Eine Absenkung der Konzentration und/oder der Aktivität der ALP wirkt daher der Osteoporose entgegen. Diesen Effekt weist z. B. Testosteron auf. Durch die Messung der Expression von Proteinen (z. B. ALP, FAK) wird indirekt deren Konzentration gemessen, da diese wesentlich vom Ausmaß der Expression abhängt. Je stärker die Expression, umso höher ist die Konzentration. Dem Fachmann sind diese Zusammenhänge bekannt, ebenso weiß er wie Untersuchungen der Expression von Enzymen, und somit auch der ALP, quantitativ durchgeführt werden, so dass darauf hier nur allgemein eingegangen werden muss. Die Expression der Alkalischen Phosphatase (und damit in bekannter Korrelation deren Konzentration) wird mit Hilfe von fluoreszenzbasierten Nachweisreaktionen (Immunofluoreszenzmessungen) bestimmt, für deren Durchführung die erforderlichen Chemikalien und Geräte kommerziell erhältlich sind. Dem Fachmann ist die Handhabung und der Einsatz dieser Chemikalien und Geräte, sowie auch der sonstigen allgemein üblichen und erforderlichen Laborausrüstung für Immunofluoreszenzmessungen bekannt. Im Rahmen der vorliegenden Ausführungsbeispiele (Beispielmessungen) wurden alle dem Fachmann bekannten Parameter, beispielsweise Lösungsmittel- und Pufferzusammensetzungen sowie -Konzentrationen, Temperaturen, Geräteeinstellungen (z. B. für die Messung der Fluoreszenzstärke) usw. in geeigneten Grenzen konstant gehalten, so dass die Versuchsergebnisse die beschriebenen quantitativ-vergleichbaren relativen Ergebnisse liefern. Die Mitführung von Testosteron bei allen durchgeführten Messungen dient sozusagen als "interner Standard" gegenüber der Messung der Proteine und der jeweils unter gleichen Bedingungen ebenfalls durchgeführten Kontrollmessung.
Sowohl Testosteron als auch die Tetrapeptide (Peptid-1 und Peptid-2) induzieren eine Abnahme der grünen Fluoreszenz, d.h. eine Abnahme der Expression und somit der Konzentration der Alkalischen Phosphatase in SAOS-2 Osteoblasten, was eine Überführung der SAOS-2 Osteoblasten in SAOS-2 Osteozyten anzeigt und somit einen Nachweis für die Wirksamkeit darstellt (vgl. Fig. 2A und 2B). Fig. 2C zeigt anhand einer graphischen Darstellung beispielhaft die Ergebnisse der quantitativen Auswertung der ALP-Messungen (ALP-Analysen). Die Durchführung der dafür erforderlichen Auswertungen von ALP-Messungen (Fluoreszenzintensitäten) ist dem Fachmann bekannt.
Zur eindeutigen Quantifizierung der Wirkungen der Tetrapeptide (Peptid-1 und Peptid-2) und somit der Definition des Schutzbereichs wird wie folgt vorgegangen: Dem Fachmann ist im Rahmen der Anwendung von Fluoreszenz-Assays bekannt, dass die Stärke der gemessenen Fluoreszenzsignale direkt proportional mit der Menge des anvisierten, fluoreszenz-markierten Objektes (hier die verschiedenen Proteine) korreliert. Die Fluoreszenz kann mithilfe eines Fluoreszenzmikroskops in einem möglichst engen Raster entweder punktuell oder über das gesamte Okularbild erfasst und quantifiziert werden. Das geschieht mithilfe von Mithilfe von Histogrammen welche die Häufigkeit (Frequenz) einer bestimmten Fluoreszenzintensität über das gesamte Okular-Blickfeld erfassen und ihre Darstellung bildlich ermöglichen. Die relevanten Einflussparameter sind dem Fachmann bekannt und es ist daher im Stand der Technik üblich, gleichbehandelte und in gleicher Weise vermessene Referenz- und/oder negative Kontrollproben mit zu untersuchen. In Relation zu den analogen Messergebnissen der Blindproben und/oder der Referenzproben können die Ergebnisse der Fluoreszenzmessungen daher dann quantitativ ausgewertet werden. Ein vergleichbares Vorgehen ist zum Beispiel auch in der chemischen Analytik (z. B. NMR-Spektroskopie) üblich, dort unter dem Begriff "interner Standard": Der zu vermessenden Probe wird ein der zu bestimmenden Substanz sehr ähnliche Verbindung in bekannter Menge zugegeben, beide erscheinen als Messsignale im Messergebnis, und unter Bezug auf die Signalintensität der in bekannter Menge zugegebenen ähnlichen Verbindung, kann auf die vorliegende Menge der zu bestimmenden Substanz geschlossen werden. Die hier angewendete Vorgehensweise ist dem analog, da die Blindproben (Zellen ohne Behandlung) und/oder die Referenzproben (mit Testosteron behandelte Proben) jeweils als interner Standard dienen.
Dem Fachmann ist auch bekannt, dass die niedrigen Fluoreszenz-Intensitätswerte ("Wert", z. B. jeweils auf den Abszissen der Fig. 2B und Fig. 4F) infolge der Nähe zum Untergrundrauschen unzuverlässig sind. Die Messung und Auswertung der Häufigkeit ("Frequenz", z.B. jeweils auf den Ordinaten der Fig. 2B und 4F) höherer Intensitätswerte ist daher sicherer zur Definition der erfindungsgemäßen Wirkung. In Fig. 2B und Fig. 4F ist dies für den Fachmann z. B. deutlich erkennbar an den sehr stark schwankenden Häufigkeitswerten bei niedrigen Abszissenwerten. Erst etwa ab dem Wert 45 (Fig. 2B) bzw. 65 (Fig. 4F) auf der Abszissenachse verlaufen die gemessenen Häufigkeitswerte (Ordinatenachse) nicht mehr unvorhersehbar schwankend, sondern stabil und können korrekt ausgewertet werden. Zur Definition der Wirksamkeit können daher verlässlich erst ab dem Intensitätswert 45 (Fig. 2B) bzw. 70 (Fig. 4F) gemessene Häufigkeiten verwendet werden.
Dies gilt nicht nur für die Auswertungen der Messungen bezüglich der Alkalischen Phosphatase, sondern generell für alle mittels Fluoreszenzmessungen definierten Wirkungen des Gegenstands der Erfindung.
Die Wirksamkeit der Tetrapeptide insbesondere die besondere Wirksamkeit von Tetrapeptid Seq ID 1 wird weiterhin anhand der Aktivierung der Fokalen Adhäsions Kinase (FAK) nachgewiesen. Aktivierte FAK geht der Bildung von Myotuben (Muskelfasern) voraus. Diese Messungen erfolgen analog zu den Messungen der ALP, mit den hierfür (FAK-Messung) erforderlichen Chemikalien und Geräten. Auch hierbei wird eine grüne Fluoreszenz entsprechender Versuchsansätze in der dem Fachmann bekannten Art und Weise gemessen und ausgewertet, um quantitativ vergleichbare relative Werte zu erhalten; das weiter oben bezüglich ALP Gesagte gilt hierfür analog. Für diese Untersuchungen (Expression/Konzentration der FAK) werden Versuche unter Verwendung kommerziell erhältlicher Myoblasten (Zelllinie L6) durchgeführt, wobei auch hierbei wiederum Testosteron als Referenzsubstanz verwendet wird. L6-Myoblasten exprimieren ZIP9-Gensequenzen, aber nicht den klassischen Androgen-Rezeptor, woraus erkennbar ist, dass die Peptide auch mit dem daraus exprimierten Rezeptor wechselwirken.
Die Abbildungen Fig. 3A und Fig. 3B zeigen beispielhaft die Ergebnisse dieser Messungen: Nach 6 Stunden Inkubation sieht man eine stärkere grüne Fluoreszenz (hellere Grauschattierung) in Testosteron- oder Peptid-1-behandelten Zellen gegenüber der Referenzprobe (Fig. 3A). Nach 9 Stunden Inkubation hat sich in der Kontrollprobe ("Kontrolle") nichts geändert, wohingegen Testosteron- und Peptid-1-behandelte L6-Myoblasten Reihen von verschmolzenen Zellen, sogenannten Myotuben (= Muskelfasern) gebildet haben (Fig. 3B, gekennzeichnet mit dem Bezugszeichen "10"), welche durch den Nachweis von darin aktivierter FAK sichtbar gemacht werden. Dies stützt beispielhaft die Wirksamkeit der Peptide zur Stimulierung des Muskelaufbaus.
Die Wirksamkeit der Tetrapeptide, insbesondere die Wirksamkeit von Tetrapeptid Seq ID 1 wird weiterhin anhand der stimulierenden Wirkung der Peptide auf die Expression von Zonula Occludens-1 (ZO-1) in Sertoli-Zellen nachgewiesen, wobei auch hierbei wiederum Testosteron als Referenzsubstanz verwendet wird (vgl. Fig. 4A-F). ZO-1 ist eins von mehreren Proteinen der Blut-Hoden-Schranke, so dass diese Versuche beispielhaft die Wirksamkeit der Peptide zur Stimulierung der Expression von Proteinen, welche für den Aufbau der Blut-Hoden-Schranke zuständig sind, stützen. Auch für die Untersuchung der Expression von ZO-1 in Sertoli-Zellen sind dem Fachmann die Vorgehensweise und Durchführung bekannt. Diese Messungen erfolgen ebenfalls analog zu den Messungen der ALP, mit den hierfür (ZO-1-Messung) erforderlichen Chemikalien und Geräten. Auch hierbei wird eine grüne Fluoreszenz entsprechender Versuchsansätze in der dem Fachmann bekannten Art und Weise gemessen und ausgewertet, um quantitativ vergleichbare relative Werte zu erhalten; das weiter oben bezüglich ALP Gesagte gilt hierfür analog.

### Abkürzungsverzeichnis

- ALP: **AL**kalischen **P**hosphatase (Enzym)
- AR: **A**ndrogen-**R**ezeptor
- ZO-1: **Z**onula **O**ccludens-**1** (Enzym)
- SAOS-2: **SA**rcoma **OS**teogenic-**2** (Zellinie)
- FAK: **F**okale **A**dhäsions**k**inase (Enzym)
- ZIP-9: **Z**ink Transporter 9

### Abbildungslegenden und Bezugszeichenliste

- Fig. 1A:: Nachweis der Stimulierung der Ossifikation in SAOS-2-Zellen durch Testosteron (als Vergleichswert) und der erfindungsgemäßen Tetrapeptide (Peptid-1 und Peptid-2) nach 4 Tagen Inkubation.
- Fig. 1B:: Nachweis der Stimulierung der Ossifikation in SAOS-2-Zellen durch Testosteron (als Vergleichswert) und der erfindungsgemäßen Tetrapeptide (Peptid-1 und Peptid-2) nach 11 Tagen Inkubation.
- Fig. 2A:: Nachweis der Aktivität der ALP anhand der Fluoreszenz in SAOS-2-Zellen. Vergleichsmessungen, beispielhaft durchgeführt an Peptid-1 und Peptid-2, im Vergleich zu Testosteron sowie einer Referenzmessung ohne wirksame Zusätze; Zellkerne sind dunkel eingefärbt.
- Fig. 2B:: Beispielhafte Darstellung der quantitativen Auswertung der Bestimmung des Grades der ALP-Hemmung/Reduktion. Linie 1 zeigt die graphische Darstellung der quantitativen Auswertung der Kontrollversuche (unbehandelte Zellen). Linie 2 zeigt die graphische Darstellung der quantitativen Auswertung der Vergleichsversuche (mit Testosteron behandelte Zellen). Linie 3 zeigt die graphische Darstellung der quantitativen Auswertung der Ausführungsbeispiele mit Tetrapeptid 1 Seq ID 1 (Peptid-1). Linie 4 zeigt die graphische Darstellung der quantitativen Auswertung der erfindungsgemäßen Ausführungsbeispiele mit Die Tetrapeptid 2 Seq ID 2 (Peptid-).
- Fig. 3A:: Beispielhaft ausgewählte FAK-Messung an mit Tetrapeptid 1 Seq ID 1 (Peptid-1) behandelten L6-Myoblasten (Ergebnis nach 6 h Inkubation)
- Fig. 3B:: Beispielhaft ausgewählte FAK-Messung an mit Tetrapeptid 1 Seq ID 1 (Peptid-1) behandelten L6-Myoblasten (Ergebnis nach 9 h Inkubation).
- Fig. 4A:: Beispielhafte Darstellung eines Kontrollversuchs zum Nachweis der stimulierenden Wirkung der Tetrapeptide auf die Expression von Zonula occludens-1 (ZO-1) in Sertoli-Zellen.
- Fig. 4B:: Beispielhafte Darstellung eines Versuchs mit Peptid-1 zum Nachweisl der stimulierenden Wirkung der Tetrapeptide auf die Expression von Zonula occludens-1 (ZO-1) in Sertoli-Zellen.
- Fig. 4C:: Beispielhafte Darstellung eines Versuchs mit Peptid-2 zum Nachweis der stimulierenden Wirkung der Tetrapeptide auf die Expression von Zonula Occludens-1 (ZO-1) in Sertoli-Zellen.
- Fig. 4D:: Beispielhafte Darstellung eines Versuchs mit Peptid-3 zum Nachweis der stimulierenden Wirkung der Tetrapeptide auf die Expression von Zonula occludens-1 (ZO-1) in Sertoli-Zellen.
- Fig. 4E:: Beispielhafte Darstellung eines Versuchs mit Testosteron als Vergleich zur stimulierenden Wirkung der Tetrapeptide auf die Expression von Zonula occludens-1 (ZO-1) in Sertoli-Zellen.
- Fig. 4F:: Beispielhafte Darstellung der quantitativen Auswertung der Versuche gemäß Fig. 4A-E, d.h. der stimulierenden Wirkung der Tetrapeptide auf die Expression von Zonula occludens-1 (ZO-1) in Sertoli-Zellen.
- Fig. 4G:: Exemplarische Teildarstellung von Fig. 4F, worin der Überschaubarkeit halber eine Kurve ausgelassen wurde, damit die Verläufe der in der Abbildung verbliebenen Kurven deutlicher hervortreten, und auch der Verlauf der ausgelassenen Kurve im Vergleich zu Fig. 4F, Fig. 4H, Fig. 4I besser erkennbar ist. Hier ausgelassen: Verlauf der Kurve 5 (Peptid-3).
- Fig. 4H:: Exemplarische Teildarstellung von Fig. 4F, worin der Überschaubarkeit halber eine Kurve ausgelassen wurde, damit die Verläufe der in der Abbildung verbliebenen Kurven deutlicher hervortreten, und auch der Verlauf der ausgelassenen Kurve im Vergleich zu Fig. 4F, Fig. 4G, Fig. 4I besser erkennbar ist. Hier ausgelassen: Verlauf der Kurve 3 (Peptid-1).
- Fig. 4I:: Exemplarische Teildarstellung von Fig. 4F, worin der Überschaubarkeit halber eine Kurve ausgelassen wurde, damit die Verläufe der in der Abbildung verbliebenen Kurven deutlicher hervortreten, und auch der Verlauf der ausgelassenen Kurve im Vergleich zu Fig. 4F, Fig. 4G, Fig. 4H besser erkennbar ist. Hier ausgelassen: Verlauf der Kurve 4 (Peptid-2).

Linie 1 zeigt die graphische Darstellung der quantitativen Auswertung der Kontrollversuche (unbehandelte Zellen).
Linie 2 zeigt die graphische Darstellung der quantitativen Auswertung der Vergleichsversuche (mit Testosteron behandelte Zellen).
Linie 3 zeigt die graphische Darstellung der quantitativen Auswertung der Ausführungsbeispiele mit Tetrapeptid 1 Seq ID 1 (Peptid-1).
Linie 4 zeigt die graphische Darstellung der quantitativen Auswertung der Ausführungsbeispiele mit Tetrapeptid 2 Seq ID 2 (Peptid-2).
Linie 5 zeigt die graphische Darstellung der quantitativen Auswertung der Ausführungsbeispiele mit Tetrapeptid 3 Seq ID 3 (Peptid-3).
Linie 10 zeigt im Rahmen der Ausführungsbeispiele bzw. der Vergleichsversuche gebildete Myotuben.

Gleiche Bezugszeichen haben in den verschiedenen Figuren die gleiche Bedeutung.

## Patentansprüche

1. Tetrapeptid, bestehend aus vier Aminosäuren, die mittels Peptidbindungen chemisch miteinander verknüpft sind, **dadurch gekennzeichnet, dass** es die Aminosäuren IAPG (Seq ID 1) aufweist und an den Androgenrezeptor ZIP-9 bindet.

2. Tetrapeptid gemäß Anspruch 1, **dadurch gekennzeichnet, dass** die Aminosäuren L-Aminosäuren sind.

3. Tetrapeptid gemäß Anspruch 1, **dadurch gekennzeichnet, dass** die Aminosäuren D-Aminosäuren sind.

4. Medizinisches Mittel zur Behandlung von Osteoporose, myodegenerativen Erkrankungen sowie der männlichen Infertilität eines Menschen oder Tieres umfassend ein Tetrapeptid gemäß einem der Ansprüche 1 bis 3.

5. Medizinisches Mittel gemäß Anspruch 4, **dadurch gekennzeichnet, dass** es einem Menschen oder Tier oral, intramuskulär, intraossär, rektal, parenteral, intravenös, subkutan, intracisternal, intravaginal, intraperitoneal, intrathekal, intravasculär, lokal als Puder, Salbe oder Tropfen, in Sprayform oder als Pflaster verabreicht wird.

6. Medizinisches Mittel gemäß Anspruch 4 und 5 **dadurch gekennzeichnet, dass** es einen physiologisch akzeptablen Trägerstoff, Konservierungsmittel, Puffern oder Treibmittel umfasst.

7. Medizinisches Mittel gemäß Anspruch 4 bis 6 **dadurch gekennzeichnet, dass** es zur Therapie der Osteoporose, von myodegenerativen Erkrankungen sowie der männlichen Infertilität verwendet wird.

8. Medizinisches Mittel gemäß Anspruch 4 bis 6 **dadurch gekennzeichnet, dass** es die Ossifikation in Osteoblasten stimuliert.

9. Medizinisches Mittel gemäß Anspruch 4 bis 6 **dadurch gekennzeichnet, dass** es die Expression der Alkalischen Phosphatase in Zellen, die den ZIP-9 Rezeptor aufweisen, reduziert.

10. Medizinisches Mittel gemäß Anspruch 4 bis 6 **dadurch gekennzeichnet, dass** es die Expression der Fokalen Adhäsionskinase in Zellen, die den ZIP-9 Rezeptor aufweisen, stimuliert.

11. Medizinisches Mittel gemäß Anspruch 4 bis 6 **dadurch gekennzeichnet, dass** es die Expression von Zona Occudens-1 in Zellen, die den ZIP-9 Rezeptor aufweisen, stimuliert.

12. Medizinisches Mittel gemäß Anspruch 4 bis 6 **dadurch gekennzeichnet, dass** es die Expression von Myogenin in Zellen, die den ZIP-9 Rezeptor aufweisen, stimuliert.

13. Medizinisches Mittel gemäß Anspruch 4 bis 6 **dadurch gekennzeichnet, dass** es die Expression von Claudin-5 in Zellen, die den ZIP-9 Rezeptor aufweisen, stimuliert.

14. Verwendung eines Tetrapeptides gemäß einem der Ansprüche 1 bis 3 als Nahrungsergänzungsmittel.

15. Verwendung eines Tetrapeptides gemäß einem der Ansprüche 1 bis 3 als Futterergänzungsmittel in der Tierhaltung zum Aufbau von Muskelmasse.

16. Verwendung eines Tetrapeptides gemäß einem der Ansprüche 1 bis 3 zur in vitro Züchtung von Muskelmasse.
